# EUROPEAN PATENT APPLICATION

(11) **EP 3 831 370 A1**
(43) Date of publication of application: **09.06.2021**
(21) Application number: 19842105.9
(22) Date of filing: 26.07.2019
(51) Int. Cl.: A61K 31/137, A61P 35/00, A61P 35/02

(54) **AGENT FOR INHIBITING RECURRENCE OF HEMATOLOGICAL MALIGNANCY IN PATIENTS WHO HAVE UNDERGONE HEMATOPOIETIC STEM CELL TRANSPLANTATION**

(30) Priority: 27.07.2018 JP 2018141411; 28.02.2019 JP 2019036598
(71) Applicant: Priothera Limited, Dublin 2 (IE)
(72) Inventor: CORRADO Claudia, 01710, Thoiry (FR); BUCHER Christoph, 4402, Frenkendorf (CH); JONES Julie, 4002, Basel (CH); GERGELY Peter, 4002, Basel (CH)
(74) Representative: Plasseraud IP
(86) International application number: PCT/JP2019/029524
(87) International publication number: WO 2020/022507

(57) **Abstract**

A pharmaceutical preparation that inhibits recurrence of hematological malignancies and / or improves survival rates, in patients who have undergone hematopoietic stem cell transplantation for the treatment of hematological malignancies, which contains 2-amino-2- [4-(3-benzyloxyphenylthio) -2-chlorophenyl] ethyl-propane-1,3-diol or a pharmaceutically acceptable salt thereof as an active ingredient.

## Description

### [Technical Field]

The present invention relates to a pharmaceutical preparation for inhibiting the recurrence of a hematological malignancy and improving the survival rate in a patient who has undergone hematopoietic stem cell transplantation for the treatment of a hematological malignancy.

### [Background Art]

Hematopoietic stem cell transplantation may be performed in patients suffering from hematological malignancies that are difficult to be cured with normal chemotherapies, for the treatment of hematological malignancies. However, in the patients who have undergone hematopoietic stem cell transplantation, the recurrence of hematologic malignancies may occur and the survival rates thereof may be further improved.

2-Amino-2- [4- (3-benzyloxyphenylthio) -2-chlorophenyl] ethyl-propane-1,3-diol (international nonproprietary name: moclavimod) and salts thereof correspond to a sphingosine-1-phosphate-receptor agonist, and it is known that they have immunoinhibitive effects (Patent Document 1). It is reported that the compounds mentioned above have been used for the treatment of hepatitis (Patent Document 2), for the treatment of inflammatory bowel diseases (Patent Document 3), and for the prevention of graft-versus-host diseases (GvHD) after hematopoietic stem cell transplantation (Patent Document 4), for promoting graft survival after hematopoietic stem cell transplantation (Patent Document 5), and the like.

However, there is no report in which 2-amino-2- [4- (3-benzyloxyphenylthio) -2-chlorophenyl] ethyl-propane-1,3-diol and salts thereof are used for inhibiting the recurrence of hematological malignancies and improving the survival rates in patients who have undergone hematopoietic stem cell transplantation for the treatment of hematological malignancies. In addition, it is not known that S1P receptor agonists or other immunosuppressants have such effects.

### [Prior Art Documents]

### [Patent Documents]

[Patent Document 1]
   PCT International Publication No. WO 03/029205
[Patent Document 2]
   PCT International Publication No. WO 2007/043433
[Patent Document 3]
   PCT International Publication No. WO 2007/091501
[Patent Document 4]
   PCT International Publication No. WO 2014/128611
[Patent Document 5]
   PCT International Publication No. WO 2017/153889

### [Summary of the Invention]

### [Problems to be Solved by the Invention]

It is an objective of the present invention to provide a pharmaceutical preparation that inhibits the recurrence of hematological malignancies and/or a pharmaceutical preparation that improves the survival rates, in patients who have undergone hematopoietic stem cell transplantation for the treatment of hematological malignancies.

### [Means for Solving the Problems]

The inventors of the present application discovered that a pharmaceutical preparation containing 2-amino-2- [4- (3-benzyloxyphenylthio) -2-chlorophenyl] ethyl-propane-1,3-diol or a pharmaceutically acceptable salt thereof can achieve the objective mentioned above. Thereby, the present invention has been completed. That is, the present invention includes the following aspects.
[1] A pharmaceutical preparation that inhibits recurrence of hematological malignancies in patients who have undergone hematopoietic stem cell transplantation for the treatment of hematological malignancies, which includes 2-amino-2- [4- (3-benzyloxyphenylthio) -2-chlorophenyl] ethyl-propane-1,3-diol or a pharmaceutically acceptable salt thereof as an active ingredient.
[2] A pharmaceutical preparation that improves survival rates in patients who have undergone hematopoietic stem cell transplantation for the treatment of hematological malignancies, which includes 2-amino-2-[4- (3-benzyloxyphenylthio) -2-chlorophenyl] ethyl-propane-1,3-diol or a pharmaceutically acceptable salt thereof as an active ingredient.
[3] The pharmaceutical preparation as recited in [1] or [2], which is used together with one or more other immunosuppressants.
[4] The pharmaceutical preparation as recited in any one of [1] to [3], wherein a dosage of 2-amino-2- [4- (3-benzyloxyphenylthio) -2-chlorophenyl] ethyl-propane-1,3-diol or a pharmaceutically acceptable salt thereof is 1 to 3 mg / day in terms of 2-amino-2- [4- (3-benzyloxyphenylthio) -2-chlorophenyl] ethyl-propane-1,3-diol.
[5] The pharmaceutical preparation as recited in any one of [1] to [4], wherein the hematological malignancy is acute myeloid leukemia or acute lymphocytic leukemia.
[6] The pharmaceutical preparation as recited in any one of [1] to [5], which is used for a patient for 80 days or more.
[7] The pharmaceutical preparation as recited in any one of [1] to [6], wherein the dosage of 2-amino-2- [4- (3-benzyloxyphenylthio) -2-chlorophenyl] ethyl-propane-1,3-diol or a pharmaceutically acceptable salt thereof is 3 mg / day in terms of 2-amino-2- [4- (3-benzyloxyphenylthio) -2-chlorophenyl] ethyl-propane-1,3-diol, and is used together with methotrexate and cyclosporin A.
[8] 2-Amino-2- [4- (3-benzyloxyphenylthio) -2-chlorophenyl] ethyl-propane-1,3-diol or a pharmaceutically acceptable salt thereof for use in inhibiting the recurrence of hematologic malignancies in patients who have undergone hematopoietic stem cell transplantation for the treatment of hematologic malignancies.
[9] 2-amino-2- [4- (3-benzyloxyphenylthio) -2-chlorophenyl] ethyl-propane-1,3-diol or a pharmaceutically acceptable salt thereof for use in improving the survival rate of patients who have undergone hematopoietic stem cell transplantation for the treatment of hematological malignancies.
[10] The compound or a pharmaceutically acceptable salt thereof, as recited in [8] or [9], which is used together with one or more other immunosuppressants.
[11] The compound or a pharmaceutically acceptable salt thereof, as recited in any one of [8] to [10], wherein the dosage of 2-amino-2-[4- (3-benzyloxyphenylthio) -2-chlorophenyl] ethyl-propane-1,3-diol or a pharmaceutically acceptable salt thereof is 1 to 3 mg / day in terms of 2-amino-2- [4- (3-benzyloxyphenylthio) -2-chlorophenyl] ethyl-propane-1,3-diol.
[12] The compound or a pharmaceutically acceptable salt thereof as recited in any one of [8] to [11], wherein the hematological malignancy is acute myeloid leukemia or acute lymphocytic leukemia.
[13] The compound or a pharmaceutically acceptable salt thereof as recited in any one of [8] to [12], which is used for a patient for 80 days or more.
[14] The compound or a pharmaceutically acceptable salt thereof as recited in any one of [8] to [13], wherein the dosage of 2-amino-2-[4- (3-benzyloxyphenylthio) -2-chlorophenyl] ethyl-propane-1,3-diol or a pharmaceutically acceptable salt thereof is 3 mg / day in terms of 2-amino-2- [4- (3-benzyloxyphenylthio) -2-chlorophenyl] ethyl-propane-1,3-diol, and is used together with methotrexate and cyclosporin A.
[15] A method for inhibiting the recurrence of hematological malignancies, comprising administering 2-amino-2- [4- (3-benzyloxyphenylthio) -2-chlorophenyl] ethyl-propane-1,3-diol or a pharmaceutically acceptable salt thereof, to patients who have undergone hematopoietic stem cell transplantation for the treatment of hematological malignancies.
[16] A method for improving survival rates of patients who have undergone hematopoietic stem cell transplantation for the treatment of hematological malignancies, the method comprising administering 2-amino-2- [4- (3-benzyloxyphenylthio) -2-chlorophenyl] ethyl-propane-1,3-diol or a pharmaceutically acceptable salt thereof to the patients.
[17] The method as recited in [15] or [16], wherein 2-amino-2-[4- (3-benzyloxyphenylthio) -2-chlorophenyl] ethyl-propane-1,3-diol or a pharmaceutically acceptable salt thereof is administered together with one or more other immunosuppressants.
[18] The method as recited in any one of [15] to [17], wherein the dosage of 2-amino-2- [4- (3-benzyloxyphenylthio) -2-chlorophenyl] ethyl-propane-1,3-diol or a pharmaceutically acceptable salt thereof is 1 to 3 mg / day in terms of 2-amino-2- [4- (3-benzyloxyphenylthio) -2-chlorophenyl] ethyl-propane-1,3-diol.
[19] The method as recited in any one of [15] to [18], wherein the hematological malignancy is acute myeloid leukemia or acute lymphocytic leukemia.
[20] The method as recited in any one of [15] to [19], wherein the method is carried out for a patient for 80 days or more.
[21] The method as recited in any one of [15] to [20], wherein the dosage of 2-amino-2- [4- (3-benzyloxyphenylthio) -2-chlorophenyl] ethyl-propane-1,3-diol or a pharmaceutically acceptable salt thereof is 3 mg / day in terms of 2-amino-2- [4- (3-benzyloxyphenylthio) -2-chlorophenyl] ethyl-propane-1,3-diol, and is used together with methotrexate and cyclosporin A.

### [Effects of the Invention]

According to the present invention, a pharmaceutical preparation for inhibiting the recurrence of hematological malignancies in patients who have undergone hematopoietic stem cell transplantation for the treatment of hematological malignancies can be provided. In addition, a pharmaceutical preparation for improving the survival rates of patients who have undergone hematopoietic stem cell transplantation for the treatment of hematological malignancies can be provided.

### [Modes for Carrying out the Invention]

In the present embodiments, "2-amino-2- [4- (3-benzyloxyphenylthio) -2-chlorophenyl] ethyl-propane-1,3-diol or a pharmaceutically acceptable salt thereof" can be produced by the methods disclosed in, for example, WO 03/029184 and WO 2006/041019.

In the present embodiments, as examples of the "pharmaceutically acceptable salt thereof", mention may be made of, for example, acid addition salts such as hydrochloride, hydrobromide, sulfate, phosphate, acetate, trifluoroacetate, citrate, tartrate, methanesulfonate, p-toluenesulfonate and the like, of 2-amino-2- [4- (3-benzyloxyphenylthio) -2-chlorophenyl] ethyl-propane-1,3-diol. Among these, a hydrochloride of 2-amino-2- [4- (3-benzyloxyphenylthio) -2-chlorophenyl] ethyl-propane-1, 3-diol is particularly preferable.

In the present embodiments, the daily dosage of 2-amino-2- [4-(3-benzyloxyphenylthio) -2-chlorophenyl] ethyl-propane-1,3-diol or a pharmaceutically acceptable salt thereof can be appropriately selected depending on the weight, age, health conditions and the like of the patient. For example, the dosage preferably ranges from 0.1 to 30 mg / day, more preferably ranges from 0.5 to 30 mg / day, still more preferably ranges from 1 to 3 mg / day, and is, in particular, 3 mg / day in terms of 2-amino-2- [4- (3-benzyloxyphenylthio) -2-chlorophenyl] ethyl-propane-1,3-diol.

In the present embodiments, the pharmaceutical preparation containing 2-amino-2- [4- (3-benzyloxyphenylthio) -2-chlorophenyl] ethyl-propane-1,3-diol or a pharmaceutically acceptable salt thereof can contain a pharmaceutically acceptable carrier, an excipient, a binder, a diluent, and the like.

In the present embodiments, the pharmaceutical preparation containing 2-amino-2- [4- (3-benzyloxyphenylthio) -2-chlorophenyl] ethyl-propane-1,3-diol or a pharmaceutically acceptable salt thereof can be formulated into a dosage form such as powders, granules, tablets, capsules, solutions, suppositories, and injections, based on ordinary knowledge in the field to which the present invention pertains. Tablets, capsules, and injections, in particular, are preferable.

In the present embodiments, as examples of "hematological malignancies", mention may be made of, for example, acute myeloid leukemia (AML), chronic myeloid leukemia (CML), acute lymphocytic leukemia (ALL), chronic lymphocytic leukemia (CLL), myelodysplasia syndrome (MDS), multiple myeloma (MM), B-cell lymphoma (B-lym), myelofibrosis (MF), and the like. The pharmaceutical preparation containing 2-amino-2- [4- (3-benzyloxyphenylthio) -2-chlorophenyl] ethyl-propane-1,3-diol or a pharmaceutically acceptable salt thereof particularly exhibits the remarkable effects of improving the survival rates in patients who have undergone hematopoietic stem cell transplantation for the treatment of hematological malignancies, in the case of acute myeloid leukemia or acute lymphocytic leukemia.

In the present embodiments, the age of the patient is not particularly limited as long as the age is the target age for normal hematopoietic stem cell transplantation. The age of the patient is preferably 18 or more years old when the patient is subjected to hematopoietic stem cell transplantation.

In the present embodiments, the "inhibiting of the recurrence of hematological malignancies" means that the rate of recurrence of hematological malignancies is reduced in the group of patients to whom the pharmaceutical preparation containing 2-amino-2- [4- (3-benzyloxyphenylthio) -2-chlorophenyl] ethyl-propane-1,3-diol or a salt thereof is administered, as compared with the recurrence rate of hematologic malignancies in all groups of patients who have undergone hematopoietic stem cell transplantation for the treatment of hematologic malignancies, or as compared with the recurrence rate of hematologic malignancies in the group of patients to whom the pharmaceutical preparation containing 2-amino-2- [4- (3-benzyloxyphenylthio) -2-chlorophenyl] ethyl-propane-1,3-diol or a pharmaceutically acceptable salt thereof is not administered among all the groups of the patients mentioned above. The recurrence rate of hematologic malignancies can be calculated, for example, from the number of patients with the recurrence of malignant tumors relative to the total number of patients who have undergone hematopoietic stem cell transplantation, at the time of passing the determined days after the patient subjected to hematopoietic stem cell transplantation (for example, half a year, one year, 1.5 years, or two years). In addition, the recurrence rate can also be calculated by the Kaplan-Meier method.

In the present embodiments, the "improvement in the survival rate" means that the survival rate is increased in the group of patients to whom the pharmaceutical preparation containing 2-amino-2- [4- (3-benzyloxyphenylthio) -2-chlorophenyl] ethyl-propane-1,3-diol or a salt thereof is administered, as compared with the survival rate in all groups of patients who have undergone hematopoietic stem cell transplantation for the treatment of hematologic malignancies, or as compared with the survival rate in the group of patients to whom the pharmaceutical preparation containing 2-amino-2- [4- (3-benzyloxyphenylthio) -2-chlorophenyl] ethyl-propane-1,3-diol or a pharmaceutically acceptable salt thereof is not administered among all the groups of the patients mentioned above. The survival rate can be calculated, for example, from the number of survival patients relative to the total number of patients who have undergone hematopoietic stem cell transplantation, at the time of passing the determined days after the patient underwent hematopoietic stem cell transplantation (for example, half a year, one year, 1.5 years, or two years). In addition, the recurrence rate can also be calculated by the Kaplan-Meier method.

In the present embodiments, the pharmaceutical preparation containing 2-amino-2- [4- (3-benzyloxyphenylthio) -2-chlorophenyl] ethyl-propane-1,3-diol or a pharmaceutically acceptable salt thereof can be administered to patients in combination with other immunosuppressants commonly used in hematopoietic stem cell transplantation. As examples of such an immunosuppressant, mention may be made of methotrexate (MTX), cyclosporin A (CyA), tacrolimus (Tac), mycophenolate, and the like. The pharmaceutical preparations containing 2-amino-2- [4- (3-benzyloxyphenylthio) -2-chlorophenyl] ethyl-propane-1,3-diol or a pharmaceutically acceptable salt thereof is preferably used together with methotrexate and cyclosporin A or tacrolimus. In particular, in the case of using the same together with methotrexate and cyclosporin A, the effect of inhibiting the recurrence of hematologic malignancies and the effect of improving the survival rate, in patients who have undergone hematopoietic stem cell transplantation for the treatment of hematological malignancies are remarkable. In particular, in the case where the pharmaceutical preparations containing 2-amino-2- [4- (3-benzyloxyphenylthio) -2-chlorophenyl] ethyl-propane-1,3-diol or a pharmaceutically acceptable salt thereof are used together with methotrexate and cyclosporin A, and the dosage of 2-amino-2- [4- (3-benzyloxyphenylthio) -2-chlorophenyl] ethyl-propane-1,3-diol or a pharmaceutically acceptable salt thereof is 3 mg / day in terms of 2-amino-2- [4- (3-benzyloxyphenylthio) -2-chlorophenyl] ethyl-propane-1,3-diol, the effect of inhibiting the recurrence of hematologic malignancies and the effect of improving the survival rate, in patients who have undergone hematopoietic stem cell transplantation for the treatment of hematological malignancies are even more remarkable. The daily dosage of these immunosuppressants can be appropriately selected depending on the patient's body weight, age, health conditions, and the like. Cyclosporin A can be administered intravenously or orally at an initial dosage of 2.5 mg / kg, for example, over 2 hours every 12 hours. Methotrexate can be administered, for example, intravenously or orally at an initial dosage of 10 mg / kg.

The administration of the pharmaceutical preparation containing 2-amino-2- [4- (3-benzyloxyphenylthio) -2-chlorophenyl] ethyl-propane-1,3-diol or a pharmaceutically acceptable salt thereof is started prior to the hematopoietic stem cell transplantation, and is performed for a determined period of time after the hematopoietic stem cell transplantation. For example, administration is started 11 days before the hematopoietic stem cell transplantation, and is carried out until 100 days after the hematopoietic stem cell transplantation. In particular, in the case where the administration period is set to 80 days or more, the effect of inhibiting the recurrence of hematologic malignancies and the effect of improving the survival rate in patients who have undergone hematopoietic stem cell transplantation for the treatment of hematologic malignancies are remarkably exhibited.

In the case where cyclosporin A is used together therewith, for example, on the 8^{th} day (3 days before the hematopoietic stem cell transplantation) from the start of administration of the pharmaceutical preparation containing 2-amino-2- [4- (3-benzyloxyphenylthio) -2-chlorophenyl] ethyl-propane-1,3-diol or a pharmaceutically acceptable salt thereof, the administration of cyclosporin A is started. For example, intravenous administration of cyclosporin A is carried out at an initial dosage of 2.5 mg / kg over 2 hours every 12 hours. Dosage adjustments are carried out, based on the toxicity or the concentration of cyclosporin A relative to the target trough concentration (150 to 400 mg / L). The administration of cyclosporin A can be changed to oral administration if the patient can tolerate the oral administration. The initial dosage for oral administration may be set to the current dosage for intravenous administration. The dosage of cyclosporin A is monitored at least weekly and changed to a clinically appropriate dosage.

In the case where tacrolimus is used together therewith, for example, on the 8^{th} day (3 days prior to the hematopoietic stem cell transplantation) from the start of administration of the pharmaceutical preparation containing 2-amino-2- [4- (3-benzyloxyphenylthio) -2-chlorophenyl] ethyl-propane-1,3-diol or a pharmaceutically acceptable salt thereof, the administration of tacrolimus is started. For example, the intravenous administration of tacrolimus is started at an initial dosage of 0.03 mg / kg. The subsequent dosages are determined by hospital standards and based on blood concentration monitoring. The dosage is adjusted to maintain a recommended concentration ranging from 5 to 15 ng / mL.

In the case where methotrexate is used together therewith, the dosing schedule and the dosage of methotrexate will be adapted to the hospital standards. For example, on the 11^{th} day from the start of administration of the pharmaceutical preparation containing 2-amino-2-[4- (3-benzyloxyphenylthio) -2-chlorophenyl] ethyl-propane-1,3-diol or a pharmaceutically acceptable salt thereof, 10 mg / kg of methotrexate is administered, and on the 13^{th} day and the 16^{th} day therefrom, 6 mg / kg of methotrexate is administered, respectively.

In the case where mycophenolate is used together therewith, the mycophenolate is administered according to the hospital practice. For example, 2 × 100 mg mycophenolate per day is administered after the mini Seattle-type pretreatment. The dosage is adjusted, based on the clinical side effects.

In general, conditioning regimen is performed prior to hematopoietic stem cell transplantation. The conditioning regimen is performed to inhibit the patient's immune cells, reduce the patient's tumor cells, and destroy the patient's hematopoietic function. The conditioning regimens are classified into myeloablative conditioning (MAC), reduced-intensity conditioning (RIC), and nonmyeloablative conditioning (NMA), in accordance with Reduced-Intensity Conditioning Regimen Workshop by the Center for International Blood and Marlow Transplant Research (CIBMTR). The conditioning regimen is appropriately selected in consideration of the type of hematological malignancy, the general condition of the patient, the age of the patient, and the like. For example, the following ones may be mentioned.

### (1) Myeloablative conditioning

High-dose chemotherapy, high-dose total body irradiation (TBI), or a combination thereof is carried out. As examples of chemotherapeutic agents, mention may be made of cyclophosphamide (CY), cytarabine (CA), etoposide (ETP), busulfan (BU), fludarabine (FLU), melphalan (MEL), methotrexate (MTX), cyclosporin A (CyA), and the like. For example, a treatment consisting of administration of cyclophosphamide followed by total body irradiation, a treatment consisting of administration of busulfan and cyclophosphamide and the like may be mentioned. As examples of chemotherapy and total body irradiation regimens, mention may be made of, for example:
1) Fludarabine (25 mg / m² / day × 3 days)
2) Busulfan (0.8 mg / kg / 6 hours × 2 to 4 days)
3) Cyclophosphamide (60 mg / kg / day × 2 days)
4) Total body irradiation (200 cGy x 2 times / day × 3 days)

### (2) Reduced-intensity conditioning

Reduced-intensity conditioning is performed by chemotherapy with a combination of fludarabine and an alkylating agent. Optionally, total body irradiation with a low dosage is combined. As examples of alkylating agents combined with fludarabine, mention may be made of busulfan, melphalan, cyclophosphamide, and the like. For example, a treatment consisting of administration of fludarabine and busulfan and the like can be mentioned.

### (3) Nonmyeloablative conditioning

Nonmyeloablative conditioning is performed by chemotherapy, total body irradiation with a low dosage, or a combination thereof. For example, a mini Seattle-type pretreatment consisting of administration of fludarabine or another chemotherapeutic agent (30 mg / m² / day × 3 days) followed by total body irradiation (1 × 200 cGy / day × 1 day) and the like may be mentioned.

In the present embodiments, "hematopoietic stem cell transplantation" may include both autologous transplantation and allogeneic transplantation, but is preferably allogeneic hematopoietic stem cell transplantation.

The present invention is described with Examples. It should be understood that the scope of the present invention is not limited by the Examples.

### [Examples]

The clinical tests were performed as described below

### 1. Immunosuppressants

The following four types of immunosuppressants were used in the clinical tests.
▪ 2-Amino-2- [4- (3-benzyloxyphenylthio) -2-chlorophenyl] ethyl-propane-1,3-diol hydrochloride (prepared as capsules containing 0.5, 1, 2, 3, 4, or 5 mg in terms of 2-amino-2- [4- (3-benzyloxyphenylthio) -2-chlorophenyl] ethyl-propane-1,3-diol))
▪ Methotrexate
▪ Cyclosporin A
▪ Tacrolimus

### 2. Patients

23 patients participated in the clinical tests. The patient profiles are shown in Table 1.

**[Table 1]**

| Patient No. | Years of age | Gender | Hematological malignancy |
|---|---|---|---|
| 1 | 50 | Male | Multiple myeloma |
| 2 | 39 | Female | Acute lymphocytic leukemia |
| 3 | 57 | Female | Acute lymphocytic leukemia |
| 4 | 60 | Male | Myelodysplasia syndrome |
| 5 | 49 | Female | B-cell lymphoma |
| 6 | 26 | Male | Acute myeloid leukemia |
| 7 | 50 | Female | Acute lymphocytic leukemia |
| 8 | 55 | Male | Myelodysplasia syndrome |
| 9 | 38 | Female | Acute myeloid leukemia |
| 10 | 47 | Male | Acute myeloid leukemia |
| 11 | 62 | Male | Chronic myeloid leukemia |
| 12 | 57 | Female | Myelodysplasia syndrome |
| 13 | 35 | Female | Acute lymphocytic leukemia |
| 14 | 51 | Male | Acute myeloid leukemia |
| 15 | 23 | Male | Chronic myeloid leukemia |
| 16 | 63 | Male | Acute myeloid leukemia |
| 17 | 55 | Female | Acute myeloid leukemia |
| 18 | 56 | Female | Myelofibrosis |
| 19 | 51 | Male | Acute myeloid leukemia |
| 20 | 55 | Male | Chronic myeloid leukemia |
| 21 | 60 | Male | Non-hodgkin lymphoma / Hodgkin disease |
| 22 | 35 | Male | Non-hodgkin lymphoma / Hodgkin disease |
| 23 | 50 | Male | Chronic myeloid leukemia |

### 3. Schedule

The clinical tests was carried out according to the following schedule. However, changes were made appropriately depending on the condition of the patient and the like.
A: Screening (from the -50^{th} day to the -2^{nd} day), Baseline (the - 1^{st} day)
B: Treatment with 2-amino-2- [4- (3-benzyloxyphenylthio) -2-chlorophenyl] ethyl-propane-1,3-diol hydrochloride (from the 1^{st} day to the 111^{th} day)
B': Treatment with other immunosuppressants (according to hospital standards, and the like)
C: Conditioning regimen (from the 2n^{d} day to the 10^{th} day)
D: Allogeneic hematopoietic stem cell transplantation (the 11^{th} day)
E: Follow-up (from the 12^{th} day to the 376^{th} day), additional follow-up (from the 377^{th} day)

### 4. Treatment with an immunosuppressant

A predetermined amount of 2-amino-2- [4- (3-benzyloxyphenylthio) -2-chlorophenyl] ethyl-propane-1,3-diol hydrochloride was administered to the patients once a day, as well as, other immunosuppressants were administered to the patients on a dosing schedule and dosage according to the hospital standards and the like. The daily dosage of 2-amino-2- [4- (3-benzyloxyphenylthio) -2-chlorophenyl] ethyl-propane-1,3-diol hydrochloride administered to each patient (value in terms of 2-amino-2- [4- (3-benzyloxyphenylthio) -2-chlorophenyl] ethyl-propane-1,3-diol, the same is applied to the descriptions hereinafter), the administration period of time, and the types of the other immunosuppressants are shown in Table 2 (KRP: 2-amino-2- [4- (3-benzyloxyphenylthio) -2-chlorophenyl] ethyl-propane-1,3-diol hydrochloride, MTX: methotrexate, CyA: cyclosporin A, and Tac: tacrolimus).

**[Table 2]**

| Patient No. | Immunosuppressant | KRP administration period of time |
|---|---|---|
| 1 | KRP 3 mg + MTX + CyA | 34 days |
| 2 | KRP 3 mg + MTX + CyA | 108 days |
| 3 | KRP 3 mg + MTX + CyA | 107days |
| 4 | KRP 3 mg + MTX + CyA | 35 days |
| 5 | KRP 3 mg + MTX + CyA | 111 days |
| 6 | KRP 3 mg + MTX + CyA | 107 days |
| 7 | KRP 3 mg + MTX + CyA | 98 days |
| 8 | KRP 3 mg + MTX + CyA | 111 days |
| 9 | KRP 3 mg + MTX + CyA | 86 days |
| 10 | KRP 3 mg + MTX + CyA | 111 days |
| 11 | KRP 1 mg + MTX + CyA | 111 days |
| 12 | KRP 3 mg + MTX + Tac | 79 days |
| 13 | KRP 1 mg + MTX + CyA | 43 days |
| 14 | KRP 1 mg + MTX + CyA | 111 days |
| 15 | KRP 3 mg + MTX + Tac | 111 days |
| 16 | KRP 3 mg + MTX + Tac | 117 days |
| 17 | KRP 3 mg + MTX + Tac | 111 days |
| 18 | KRP 3 mg + MTX + Tac | 111 days |
| 19 | KRP 1 mg + MTX + CyA | 24 days |
| 20 | KRP 1 mg + MTX + CyA | 111 days |
| 21 | KRP 3 mg + MTX + Tac | 62 days |
| 22 | KRP 3 mg + MTX + Tac | 107 days |
| 23 | KRP 1 mg + MTX + CyA | 111 days |

### 5. Results

The conditions of patients who underwent allogeneic hematopoietic stem cell transplantation were subjected to following up for a certain period of time. The results are shown in Table 3. In the table, the follow-up period, the number of days of recurrence of hematological malignancies and the number of days of death represent the number of days passing from the day when allogeneic hematopoietic stem cell transplantation was performed. The survival rate and recurrence rate were calculated in accordance with the Kaplan-Meier method.

**[Table 3]**

| Patient No. | Hematological malignancy | Immunosuppressant | KRP Administration period | Follow-up period | Yes / No of recurrence of hematological malignancy | Alive / dead |
|---|---|---|---|---|---|---|
| 1 | Multiple myeloma | KRP 3 mg + MTX + CyA | 34 days | 749 days | Yes (749^{th} day) | Alive |
| 2 | Acute lymphocytic leukemia | KRP 3 mg + MTX + CyA | 108 days | 731 days | No | Alive |
| 3 | Acute lymphocytic leukemia | KRP 3 mg + MTX + CyA | 107 days | 734 days | No | Alive |
| 4 | Myelodysplasia syndrome | KRP 3 mg + MTX + CyA | 35 days | 532 days | Yes (287^{th} day) | Dead (532^{nd} day) |
| 5 | B-cell lymphoma | KRP 3 mg + MTX + CyA | 111 days | 819 days | No | Alive |
| 6 | Acute myeloid leukemia | KRP 3 mg + MTX + CyA | 107 days | 347 days | No | Alive |
| 7 | Acute lymphocytic leukemia | KRP 3 mg + MTX + CyA | 98 days | 727 days | No | Alive |
| 8 | Myelodysplasia syndrome | KRP 3 mg + MTX + CyA | 111 days | 704 days | No | Alive |
| 9 | Acute myeloid leukemia | KRP 3 mg + MTX + CyA | 86 days | 728 days | Yes (102^{nd} day) | Alive |
| 10 | Acute myeloid leukemia | KRP 3 mg + MTX + CyA | 111 days | 876 days | Yes (757^{th} day) | Dead (876^{th} day) |
| 11 | Chronic myeloid leukemia | KRP 1 mg + MTX + CyA | 111 days | 617 days | No | Alive |
| 12 | Myelodysplasia syndrome | KRP 3 mg + MTX + Tac | 79 days | 110 days | Yes (110^{th} day) | Dead (110^{th} day) |
| 13 | Acute lymphocytic leukemia | KRP 1 mg + MTX + CyA | 43 days | 326 days | Yes (252^{nd} day) | Dead (326^{th} day) |
| 14 | Acute myeloid leukemia | KRP 1 mg + MTX + CyA | 111 days | 669 days | No | Alive |
| 15 | Chronic myeloid leukemia | KRP 3 mg + MTX + Tac | 111 days | 554 days | No | Alive |
| 16 | Acute myeloid leukemia | KRP 3 mg + MTX + Tac | 117 days | 375 days | No | Alive |
| 17 | Acute myeloid leukemia | KRP 3 mg + MTX + Tac | 111 days | 246 days | Yes (98^{th} day) | Alive |
| 18 | Myelofibrosis | KRP 3 mg + MTX + Tac | 111 days | 310 days | Yes (105^{th} day) | Dead (310^{th} day |
| 19 | Acute myeloid leukemia | KRP 1 mg + MTX + CyA | 24 days | 99 days | Yes (77^{th} day) | Alive |
| 20 | Chronic myeloid leukemia | KRP 1 mg + MTX + CyA | 111 days | 193 days | No | Alive |
| 21 | Non-hodgkin lymphoma / Hodgkin disease | KRP 3 mg + MTX + Tac | 62 days | 79 days | Yes (49^{th} day) | Dead (79^{th} day |
| 22 | Non-hodgkin lymphoma / Hodgkin disease | KRP 3 mg + MTX + Tac | 107 days | 264 days | Yes (121^{st} day) | Dead (264^{th} day |
| 23 | Chronic myeloid leukemia | KRP 1 mg + MTX + CyA | 111 days | 264 days | No | Alive |

According to "Hematopoietic cell transplantation in Japan, the 2017 fiscal year, National Survey Report (the Japan Data Center for Hematopoietic Cell Transplantation / the Japan Society for Hematopoietic Cell Transplantation)", the survival rate of patients one year after hematopoietic stem cell transplantation is 70.7%. In addition, according to "the 2017 fiscal year, Results of Hematopoietic Stem Cell Transplantation in Japan (the Japan Data Center for Hematopoietic Cell Transplantation)", the survival rate of patients one year after autologous hematopoietic stem cell transplantation is 82.7%, and the survival rate of patients one year after allogeneic hematopoietic stem cell transplantation is 54.9 to 73.2%. On the other hand, from Table 3, it has become clear that in the case where the pharmaceutical preparation containing 2-amino-2- [4- (3-benzyloxyphenylthio) -2-chlorophenyl] ethyl-propane-1,3-diol or a pharmaceutically acceptable salt thereof is administered, the survival rate of the patients one year after allogeneic hematopoietic stem cell transplantation is 75%, and for this reason, the pharmaceutical preparation containing 2-amino-2- [4-(3-benzyloxyphenylthio) -2-chlorophenyl] ethyl-propane -1,3-diol or a pharmaceutically acceptable salt thereof can improve the survival rate of patients subjected to hematopoietic stem cell transplantation.

It is reported that the rate of recurrence of hematological malignancies after HLA-matched myeloablative transplantation ranges from about 25% to more than 60% (Curr Hematol Malig Rep. 2013 June; 8(2): 132 - 140). On the other hand, Table 3 shows that in the case where a pharmaceutical preparation containing 2-amino-2- [4- (3-benzyloxyphenylthio) -2-chlorophenyl] ethyl-propane-1,3-diol or a pharmaceutically acceptable salt thereof is administrated, the recurrence rate of hematologic malignancy one year after allogeneic hematopoietic stem cell transplantation was 40%.

Table 4 shows the recurrence rate of hematological malignancies and the survival rate of patients one year after allogeneic hematopoietic stem cell transplantation by each of the hematologic malignancies.

**[Table 4**

| Hematological malignancy | Number of patients | Recurrence rate | Survival rate |
|---|---|---|---|
| Acute myeloid leukemia | 7 patients | 43% | 100% |
| Acute lymphocytic leukemia | 4 patients | 25% | 75% |
| Myelodysplasia syndrome | 3 patients | 67% | 67% |
| Chronic myeloid leukemia | 4 patients | 0% | 100% |
| Multiple myeloma | 1 patient | 0% | 100% |
| B-cell lymphoma | 1 patient | 0% | 100% |
| Myelofibrosis | 1 patient | 100% | 0% |
| Non-hodgkin lymphoma / Hodgkin | 2 patients | 100% | 0% |
| disease | | | |

According to "the 2017 fiscal year, Results of Hematopoietic Stem Cell Transplantation in Japan" mentioned above, the survival rate of the patients one year after allogeneic hematopoietic stem cell transplantation is 54.9 to 73.2%. On the other hand, Table 4 shows that in the case where a pharmaceutical preparation containing 2-amino-2-[4- (3-benzyloxyphenylthio) -2-chlorophenyl] ethyl-propane-1,3-diol or a pharmaceutically acceptable salt thereof is administered, in patients with acute myeloid leukemia and acute lymphocytic leukemia, the survival rates of the patients one year after allogeneic hematopoietic stem cell transplantation are 100% and 75%, respectively, and for this reason, it has become clear that the pharmaceutical preparations containing 2-amino-2- [4- (3-benzyloxyphenylthio) -2-chlorophenyl] ethyl-propane-1,3-diol or a pharmaceutically acceptable salt thereof improve the survival rate of patients with acute myeloid leukemia and acute lymphocytic leukemia.

Table 5 shows the recurrence rate of hematological malignancies and the survival rate of the patients at one year after allogeneic hematopoietic stem cell transplantation according to each of the administration periods of the pharmaceutical preparation containing 2-amino-2- [4- (3-benzyloxyphenylthio) -2-chlorophenyl] ethyl-propane-1,3-diol or a pharmaceutically acceptable salt thereof.

**[Table 5]**

| Administration period | Number of patients | Recurrence rate | Survival rate |
|---|---|---|---|
| 100 days or more | 15 patients | 20% | 84% |
| 80 days or more | 17 patients | 24% | 86% |
| 60 days or more | 19 patients | 32% | 77% |

According to "the 2017 fiscal year, Results of Hematopoietic Stem Cell Transplantation in Japan" mentioned above, the survival rate of the patients one year after allogeneic hematopoietic stem cell transplantation is 54.9 to 73.2%. On the other hand, Table 5 shows that in the case where a pharmaceutical preparation containing 2-amino-2-[4- (3-benzyloxyphenylthio) -2-chlorophenyl] ethyl-propane-1,3-diol or a pharmaceutically acceptable salt thereof is administered for 100 days or more, for 80 days or more, or for 60 days or more, the survival rates of the patients one year after allogeneic hematopoietic stem cell transplantation are 84%, 86%, and 77%, respectively, and for this reason, it has become clear that the pharmaceutical preparations containing 2-amino-2- [4- (3-benzyloxyphenylthio) -2-chlorophenyl] ethyl-propane-1,3-diol or a pharmaceutically acceptable salt thereof improve the survival rate of patients subjected to hematopoietic stem cell transplantation.

In addition, as described above, it is reported that the rate of recurrence of hematological malignancies after HLA-matched myeloablative transplantation is about 25% to over 60%. On the other hand, Table 5 shows that in the case where a pharmaceutical preparation containing 2-amino-2- [4- (3-benzyloxyphenylthio) -2-chlorophenyl] ethyl-propane-1,3-diol or a pharmaceutically acceptable salt thereof is administered for 100 days or more, or for 80 days or more, the rates of recurrence of hematological malignancies of the patients one year after allogeneic hematopoietic stem cell transplantation are 20% and 24%, respectively, and for this reason, it has also become clear that the pharmaceutical preparations mentioned above suppress the recurrence of hematological malignancies in the patients subjected to hematopoietic stem cell transplantation.

Table 6 shows the recurrence rates of hematological malignancies and patient survival rates one year after allogeneic hematopoietic stem cell transplantation by each of the immunosuppressants.

**Table 6]**

| Immunosuppressant | Number of patients | Recurrence rate | Survival rate |
|---|---|---|---|
| KRP 3 mg + MTX + CyA | 10 patients | 20% | 100% |
| KRP 1 mg + MTX + CyA | 6 patients | 38% | 67% |
| KRP 3 mg + MTX + Tac | 7 patients | 71% | 36% |

According to "the 2017 fiscal year, Results of Hematopoietic Stem Cell Transplantation in Japan" mentioned above, the survival rate of the patients one year after allogeneic hematopoietic stem cell transplantation is 54.9 to 73.2%. On the other hand, Table 6 shows that in the case where a pharmaceutical preparation containing 3 mg of 2-amino-2- [4- (3-benzyloxyphenylthio) -2-chlorophenyl] ethyl-propane-1,3-diol or a pharmaceutically acceptable salt thereof, methotrexate and cyclosporin A are administered in combination as the immunosuppressants, the survival rate of the patients one year after allogeneic hematopoietic stem cell transplantation is 100%, and for this reason, it has become clear that the immunosuppressants improve the survival rate of patients subjected to hematopoietic stem cell transplantation.

In addition, as described above, it is reported that the rate of recurrence of hematological malignancies after HLA-matched myeloablative transplantation is about 25% to over 60%. On the other hand, Table 6 shows that in the case where a pharmaceutical preparation containing 3 mg of 2-amino-2- [4- (3-benzyloxyphenylthio) -2-chlorophenyl] ethyl-propane-1,3-diol or a pharmaceutically acceptable salt thereof, methotrexate and cyclosporin A are administered in combination as the immunosuppressants, the rate of recurrence of hematological malignancies of the patients one year after allogeneic hematopoietic stem cell transplantation is 20%, and for this reason, it has also become clear that the immunosuppressants mentioned above suppress the recurrence of hematological malignancies in the patients subjected to hematopoietic stem cell transplantation.

### [Industrial Applicability]

According to the present invention, in patients who have undergone hematopoietic stem cell transplantation for the treatment of hematological malignancies, it is possible to inhibit the recurrence of hematologic malignancies and also to improve the survival rate. For this reason, new treatment options for the treatment of hematological malignancies can be provided.

## Claims

1. A pharmaceutical preparation that inhibits recurrence of hematological malignancies in patients who have undergone hematopoietic stem cell transplantation for the treatment of hematological malignancies, which comprises 2-amino-2- [4- (3-benzyloxyphenylthio) -2-chlorophenyl] ethyl-propane-1,3-diol or a pharmaceutically acceptable salt thereof as an active ingredient.

2. A pharmaceutical preparation that improves survival rates in patients who have undergone hematopoietic stem cell transplantation for the treatment of hematological malignancies, which comprises 2-amino-2- [4- (3-benzyloxyphenylthio) -2-chlorophenyl] ethyl-propane-1,3-diol or a pharmaceutically acceptable salt thereof as an active ingredient.

3. The pharmaceutical preparation according to Claim 1 or 2, which is used together with one or more other immunosuppressants.

4. The pharmaceutical preparation according to any one of Claims 1 to 3, wherein a dosage of 2-amino-2- [4- (3-benzyloxyphenylthio) -2-chlorophenyl] ethyl-propane-1,3-diol or a pharmaceutically acceptable salt thereof is 1 to 3 mg / day in terms of 2-amino-2- [4- (3-benzyloxyphenylthio) -2-chlorophenyl] ethyl-propane-1,3-diol.

5. The pharmaceutical preparation according to any one of Claims 1 to 4, wherein the hematological malignancy is acute myeloid leukemia or acute lymphocytic leukemia.

6. The pharmaceutical preparation according to any one of Claims 1 to 5, which is used for a patient for 80 days or more.

7. The pharmaceutical preparation according to any one of Claims 1 to 6, wherein the dosage of 2-amino-2- [4- (3-benzyloxyphenylthio) -2-chlorophenyl] ethyl-propane-1,3-diol or a pharmaceutically acceptable salt thereof is 3 mg / day in terms of 2-amino-2- [4- (3-benzyloxyphenylthio) -2-chlorophenyl] ethyl-propane-1,3-diol, and is used together with methotrexate and cyclosporin A.

8. 2-Amino-2- [4- (3-benzyloxyphenylthio) -2-chlorophenyl] ethyl-propane-1,3-diol or a pharmaceutically acceptable salt thereof for use in inhibiting the recurrence of hematologic malignancies in patients who have undergone hematopoietic stem cell transplantation for the treatment of hematologic malignancies.

9. 2-Amino-2- [4- (3-benzyloxyphenylthio) -2-chlorophenyl] ethyl-propane-1,3-diol or a pharmaceutically acceptable salt thereof for use in improving the survival rate of patients who have undergone hematopoietic stem cell transplantation for the treatment of hematological malignancies.

10. The compound or a pharmaceutically acceptable salt thereof, according to Claim 8 or 9, which is used together with one or more other immunosuppressants.

11. The compound or a pharmaceutically acceptable salt thereof, according to any one of Claims 8 to 10, wherein the dosage of 2-amino-2- [4- (3-benzyloxyphenylthio) -2-chlorophenyl] ethyl-propane-1,3-diol or a pharmaceutically acceptable salt thereof is 1 to 3 mg / day in terms of 2-amino-2- [4- (3-benzyloxyphenylthio) -2-chlorophenyl] ethyl-propane-1,3-diol.

12. The compound or a pharmaceutically acceptable salt thereof according to any one of Claims 8 to 11, wherein the hematological malignancy is acute myeloid leukemia or acute lymphocytic leukemia.

13. The compound or a pharmaceutically acceptable salt thereof according to any one of Claims 8 to 12, which is used for a patient for 80 days or more.

14. The compound or a pharmaceutically acceptable salt thereof according to any one of Claims 8 to 13, wherein the dosage of 2-amino-2- [4-(3-benzyloxyphenylthio) -2-chlorophenyl] ethyl-propane-1,3-diol or a pharmaceutically acceptable salt thereof is 3 mg / day in terms of 2-amino-2- [4- (3-benzyloxyphenylthio) -2-chlorophenyl] ethyl-propane-1,3-diol, and is used together with methotrexate and cyclosporin A.

15. A method for inhibiting the recurrence of hematological malignancies, comprising administering 2-amino-2- [4- (3-benzyloxyphenylthio) -2-chlorophenyl] ethyl-propane-1,3-diol or a pharmaceutically acceptable salt thereof, to patients who have undergone hematopoietic stem cell transplantation for the treatment of hematological malignancies.

16. A method for improving survival rates of patients who have undergone hematopoietic stem cell transplantation for the treatment of hematological malignancies, the method comprising administering 2-amino-2- [4- (3-benzyloxyphenylthio) -2-chlorophenyl] ethyl-propane-1,3-diol or a pharmaceutically acceptable salt thereof to the patients.

17. The method according to Claim 15 or 16, wherein 2-amino-2- [4-(3-benzyloxyphenylthio) -2-chlorophenyl] ethyl-propane-1,3-diol or a pharmaceutically acceptable salt thereof is administered together with one or more other immunosuppressants.

18. The method according to any one of Clams 15 to 17, wherein the dosage of 2-amino-2- [4- (3-benzyloxyphenylthio) -2-chlorophenyl] ethyl-propane-1,3-diol or a pharmaceutically acceptable salt thereof is 1 to 3 mg / day in terms of 2-amino-2- [4- (3-benzyloxyphenylthio) -2-chlorophenyl] ethyl-propane-1,3-diol.

19. The method according to any one of Claims 15 to 18, wherein the hematological malignancy is acute myeloid leukemia or acute lymphocytic leukemia.

20. The method according to any one of Claims 15 to 19, wherein the method is carried out for a patient for 80 days or more.

21. The method according to any one of Claims 15 to 20, wherein the dosage of 2-amino-2- [4- (3-benzyloxyphenylthio) -2-chlorophenyl] ethyl-propane-1,3-diol or a pharmaceutically acceptable salt thereof is 3 mg / day in terms of 2-amino-2- [4- (3-benzyloxyphenylthio) -2-chlorophenyl] ethyl-propane-1,3-diol, and is used together with methotrexate and cyclosporin A.
